(19) Europäisches Patentamt
European Patent Office
Office européen des brevets

(11) Veröffentlichungsnummer: **0 282 834**
**A1**

# (12) EUROPÄISCHE PATENTANMELDUNG

(21) Anmeldenummer: **88103331.0**

(22) Anmeldetag: **04.03.88**

(51) Int. Cl.⁴: **C07C 53/128**, **A61K 31/20**

(30) Priorität: **20.03.87 DE 3709230**

(43) Veröffentlichungstag der Anmeldung:
**21.09.88 Patentblatt 88/38**

(84) Benannte Vertragsstaaten:
**AT BE CH DE ES FR GB GR IT LI LU NL SE**

(71) Anmelder: **Desitin Arzneimittel GmbH**
**Weg beim Jäger 214**
**D-2000 Hamburg 63(DE)**

(72) Erfinder: **Schäfer, Helmut, Dr.**
**Stegenerweg 40**
**D-2061 Kayhude(DE)**

(74) Vertreter: **UEXKÜLL & STOLBERG**
**Patentanwälte**
**Beselerstrasse 4**
**D-2000 Hamburg 52(DE)**

## (54) Neues Calciumsalz der Valproinsäure.

(57) Die Erfindung betrifft ein neues kristallines Calciumsalz der Valproinsäure, bei dem je Molekül 5 Valproinsäurereste mit einem Calciumion assoziiert sind. Das Salz ist nicht hygroskopisch und weist als Wirkstoff in pharmazeutischen Zubereitungen insbesondere zur oralen Applikation überlegene galenische Eigenschaften auf.

FIGUR 1

Kinetik der Wirkstoff-Freisetzung in 0,1% Natriumhydrogencarbonat-Lösung aus Tabletten von Calciumpentavalproat (+) und Natriumvalproat (Δ).

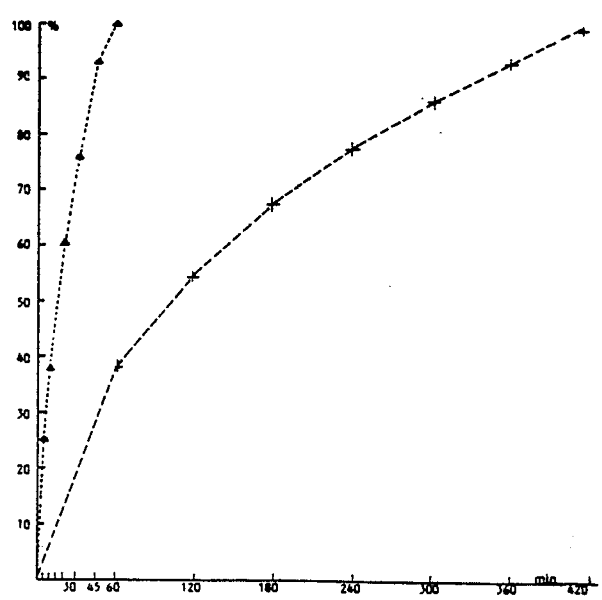


EP 0 282 834 A1

## Neues Calciumsalz der Valproinsäure

Die Erfindung betrifft ein neues Calciumsalz der Valproinsäure, welches je Molekül 5 Valproinsäurereste assoziiert mit einem Calcium-Ion enthält, sowie ein Verfahren zu dessen Herstellung.

Die pharmazeutische Wirksamkeit von Valproinsäure und deren Alkali-und Erdalkalimetallsalzen, insbesondere der Natriumsalze ist bekannt. Diese Verbindungen haben sich besonders bei der Behandlung von speziellen Epilepsieformen und von Fieberkrämpfen bewährt.

Die galenische Zubereitung dieser Verbindungen ist jedoch schwierig. Die Valproinsäure selbst ist eine ölige Flüssigkeit. Das Natriumsalz liegt zwar als kristalliner Feststoff vor, dieser ist jedoch außerordentlich hygroskopisch und zerfließt sehr schnell. Beide Verbindungen sind demgemäß nur begrenzt zur Herstellung oraler Darreichungsformen geeignet.

Es ist bereits versucht worden, diese Schwierigkeiten durch die Herstellung stabilerer, nicht hygroskopischer Alkali-oder Erdalkalisalze der Valproinsäure zu vermeiden. So sind in dem europäischen Patent 34 172 Dimere aus einem Molekül Valproinsäure und einem Molekül Natriumvalproat oder 1/2 Mol Calciumvalproat beschrieben. Die Salze werden in kristalliner Form gewonnen. Das entsprechende Calciumdihydrogenvalproat weist einen Schmelzpunkt von 175°C auf.

In der europäischen Patentanmeldung 141 267 sind polymere Calciumsalze der Valproinsäure beschrieben, die aus Monomeren bestehen, bei denen jeweils vier Valproinsäurereste mit einem Calcium-Ion assoziiert sind.

Schließlich werden in der europäischen Patentanmeldung 143 271 Metallsalze der Valproinsäure beschrieben, bei denen jeweils 3 Moleküle Valproinsäure mit einem einwertigen Metallsalz der Valproinsäure, nämlich dem Kalium-, Cäsium-oder Rubidiumsalz zusammentreten.

Bei den oben genannten, im Stand der Technik bekannten Alkali-oder Erdalkalimetallsalzen der Valproinsäure handelt es sich jeweils um solche, bei denen das Verhältnis von Alkalimetall-Ion zu Valproinsäure bzw. dem Valproatrest 1 : 2 oder 1 : 4 beträgt.

Überraschenderweise wurde erfindungsgemäß ein Calciumsalz der Valproinsäure gefunden, bei dem je Molekül 5 Valproinsäurereste mit einem Calcium-Ion assoziiert sind. Das erfindungsgemäße Salz, nachfolgend auch als Calciumpentavalproat bezeichnet, ist in Kristallform erhältlich, wobei der Schmelzpunkt der Kristalle 84 - 85°C beträgt. Die Kristalle sind nicht hygroskopisch und außerordentlich stabil. Sie lösen sich sehr gut in unpolaren Lösungsmitteln wie beispielsweise Hexan und sind jederzeit wieder aus der Lösung kristallisierbar.

Die Substanz erweist sich bei mikroskopischer Untersuchung als kristallin und einheitlich. Einkristalluntersuchungen (optisch und röntgenographisch) ergeben trikline Symmetrie und die Abmessungen der Elementarzelle. Die Morphologie besteht aus den Flächen (100), (110), (010) und (001). Bei einer wahrscheinlichen Dichte von 1.08 lassen sich zwei Formeleinheiten $Ca(C_8H_{15}O_2).[C_8H_{16}O_2]_3$ in der Elementarzelle berechnen.

Für das erfindungsgemäße Calciumpentavalproat wird die folgende hypothetische Formel angenommen:

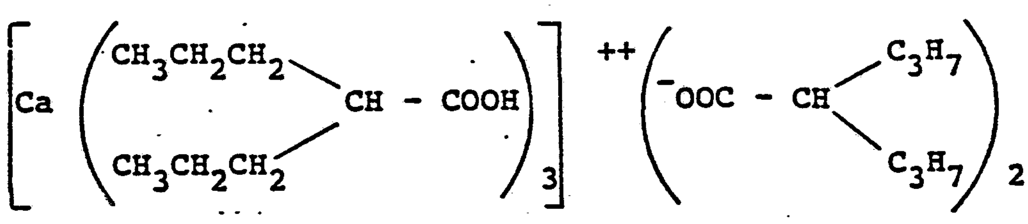

Die Erfindung ist jedoch unabhängig von dem Nachweis der Richtigkeit dieser Formel.

Das Salz kann hergestellt werden, indem man Calciumhydroxid und Valproinsäure in einem Molverhältnis von 1 : 5 in Aceton miteinander vermischt. Die Mischung wird bis zur Bildung einer im wesentlichen klaren Lösung am Rückfluß gekocht. Die Lösung wird gegebenenfalls filtriert, und nach anschließendem Abkühlen werden farblose Kristalle mit einem Schmelzpunkt von 84 - 85°C erhalten.

Die kristalline Substanz läßt sich gegebenenfalls nach Vermahlen auf eine geeignete Partikelgröße mit üblichen Trägerstoffen und Additiven leicht zu oralen Dosierungsformen wie Tabletten oder Kapseln verarbeiten.

Die mit dem erfindungsgemäßen Wirkstoff hergestellten Formulierungen weisen eine gegenüber bekannten Zubereitungen veränderte Freisetzungs-und Resorptionskinetik auf (vgl. Fig. 1).

Insbesondere läßt die stark verzögerte Freisetzung des Wirkstoffes aus den erfindungsgemäß hergestellten Calciumpentavalproat-Zubereitungen in künstlichem Darmsaft (0,1%ige Natriumhydrogencarbonat-Lösung) auf eine wünschenswerte verzögerte Resorption schließen.

Die Erfindung wird nachfolgend anhand von Beispielen erläutert:

**Beispiel 1**

Herstellung von Calciumpentavalproat

29,6 g pulverisiertes Calciumhydroxid wurden in einer Lösung von 288,4 g Valproinsäure in 1300 ml Aceton verrührt und das Gemisch 2 Stunden lang am Rückfluß gekocht.

Die fast klare Lösung wurde heiß filtriert. Aus dem Filtrat wurden nach Abkühlen auf minus 14°C durch Absaugen 206 g des erfindungsgemäßen Calciumsalzes in Form feiner, farbloser Kristalle erhalten. Der Schmelzpunkt der Kristalle betrug 84 - 85°C.

Aus der Mutterlauge wurden nach Einengen auf 1/5 des Volumens weitere 68,6 g des kristallinen Salzes gewonnen.

Die Gesamtausbeute betrug 274,6 g entsprechend 91% der Theorie.

Die nach der zweiten Kristallisation verbliebene Mutterlauge enthielt vorwiegend Valproinsäure; diese konnte zur weiteren Verwendung in dem erfindungsgemäßen Verfahren rückgeführt werden.

**Beispiel 2**

Herstellung von Tabletten

300 g des erfindungsgemäß gewonnenen Calciumvalproats wurden mit 112 g Avicel PH 102, 5 g Aerosil R 972 und 3 g Calciumstearat durch ein 0,5 mm Sieb gegeben und in einem Turbula-Mischer gemischt. Durch Abpressen auf einer Excenterpresse erhielt man Tabletten, wobei jede der Tabletten 300 mg Calciumpentavalproat enthielt.

**Beispiel 3**

Freisetzung von Valproinsäure aus Calciumpentavalproat-Tabletten

In die Körbchen eines Dissolutiontesters wurden je zwei der nach Beispiel 2 gewonnenen Tabletten gelegt. Man ließ dann bei 37°C eine unter 150 U/min gerührte 0.1%ige Natriumhydrogencarbonatlösung einwirken und entnahm, nach der 1. Stunde beginnend, in stündlichem Abstand 1000 µl-Proben aus der Bicarbonatlösung. Der Wirkstoffgehalt in den Proben wurde als "Valproinsäure" gaschromatographisch bestimmt. Die Ergebnisse sind in der nachfolgenden Tabelle wiedergegeben.

| Entnahmezeit | % freigesetzt bezogen auf 300 mg Wirkstoff-Gesamtmenge |
|---|---|
| 1 h | 38 |
| 2 h | 55 |
| 3 h | 68 |
| 4 h | 78 |
| 5 h | 86 |
| 6 h | 93 |
| 7 h | 100 |

Unter den gleichen Versuchsbedingungen wurde die Freisetzung des Wirkstoffes aus Tabletten gemessen, welche anstelle des erfindungsgemäßen Calciumpentavalproats handelsübliches Natriumvalproat enthielten. Die Probenahmen erfolgten nach 5, 10, 15, 20, 30, 45 und 60 min. Die Freisetzungskinetik des erfindungsgemäßen Calciumpentavalproats wurde derjenigen des handelsüblichen Produkts in Figur 1 gegenübergestellt.

**Ansprüche**

1. Calciumsalz der Valproinsäure mit mehr als 2 Valproinsäureresten je Calcium-Ion, **dadurch gekennzeichnet,** daß es je Molekül 5 Valproinsäurereste assoziiert mit einem Calcium-Ion enthält.

2. Verfahren zur Herstellung des Salzes nach Anspruch 1, **dadurch gekennzeichnet,** daß man eine Mischung aus Calciumhydroxid und Valproinsäure mit einem molaren Verhältnis von 1 : 5 bis zur Bildung einer im wesentlichen klaren Lösung in Aceton verkocht, die Lösung gegebenenfalls filtriert und nach Abkühlen der Lösung das Salz in kristalliner Form gewinnt.

3. Pharmazeutische Zubereitung zur Behandlung von Epilepsie und Fieberkrämpfen, **dadurch gekennzeichnet,** daß sie als Wirkstoff das Salz nach Anspruch 1 enthält.

FIGUR 1

Kinetik der Wirkstoff-Freisetzung in 0,1% Natriumhydrogencarbonat-Lösung aus Tabletten von Calciumpentavalproat (+) und Natriumvalproat (Δ).

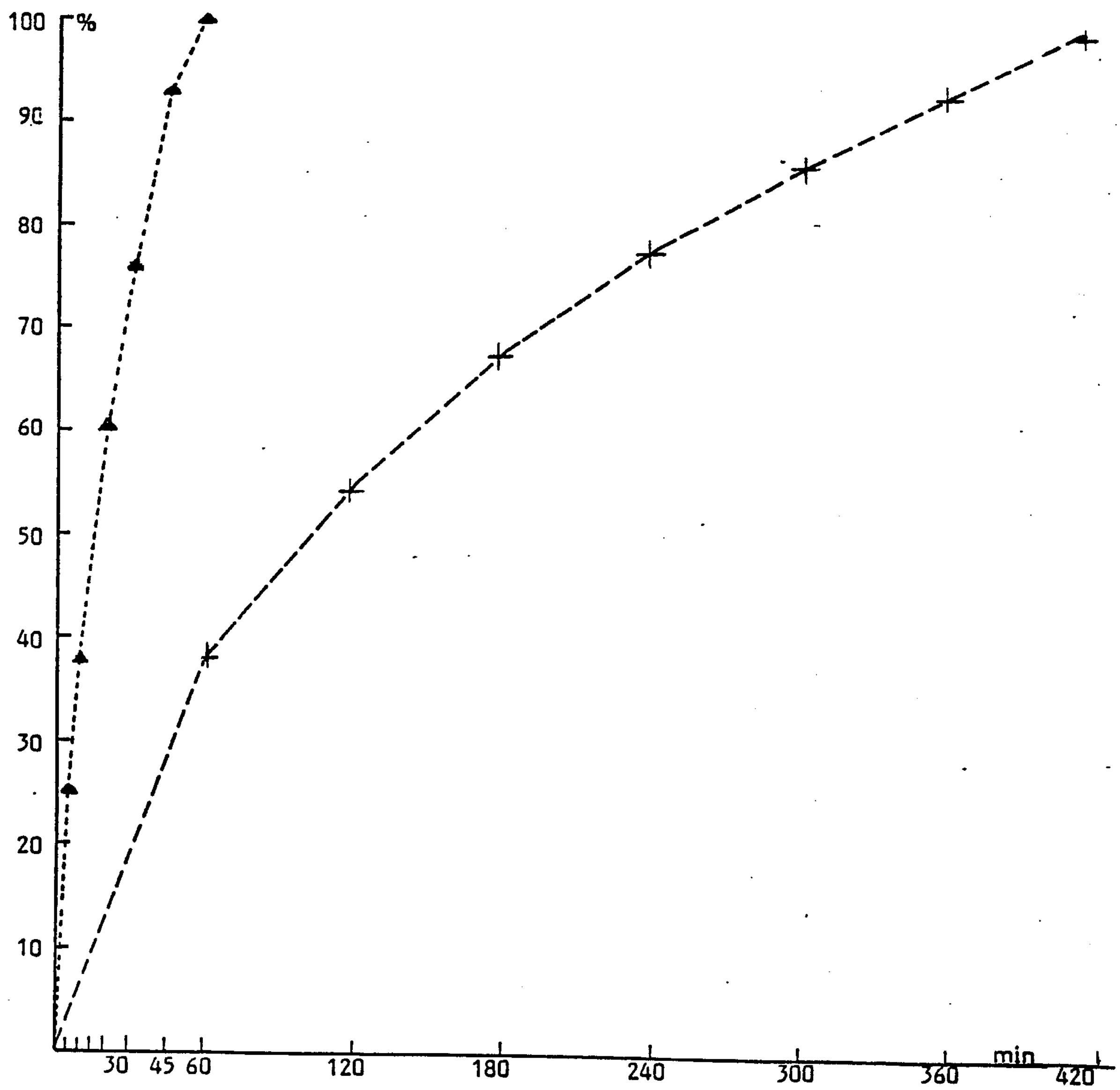

Europäisches Patentamt

# EUROPÄISCHER RECHERCHENBERICHT

Nummer der Anmeldung: EP 88103331.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| D,A | EP - A1 - 0 143 271 (ABBOTT)<br>* Beispiele; Ansprüche *<br>-- | 1-3 | C 07 C 53/128<br>A 61 K 31/20 |
| D,A | EP - A1 - 0 141 267 (ABBOTT)<br>* Beispiel; Ansprüche *<br>-- | 1-3 | |
| D,A | EP - B1 - 0 034 172 (ABBOTT)<br>* Beispiele; Ansprüche *<br>---- | 1-3 | |
| | | | RECHERCHIERTE SACHGEBIETE (Int. Cl.4) |
| | | | C 07 C 53/00 |

Der vorliegende Recherchenbericht wurde für alle Patentansprüche erstellt.

| Recherchenort | Abschlußdatum der Recherche | Prüfer |
|---|---|---|
| WIEN | 06-05-1988 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82